# EUROPEAN PATENT APPLICATION

(11) **EP 2 093 311 A2**
(43) Date of publication of application: **26.08.2009**
(21) Application number: 09152418.1
(22) Date of filing: 09.02.2009
(51) Int. Cl.: C25F 3/02

(54) **Metallic Implants**

(30) Priority: 13.02.2008 US 30689
(71) Applicant: DePuy Products, Inc., Warsaw, IN 46581 (US)
(72) Inventor: Liao, Yen-Shuo, Warsaw, IN 46582 (US); Yang, Xiaofan, Warsaw, IN 46582 (US)
(74) Representative: Belcher, Simon James

(57) **Abstract**

A method of preparing an implant comprises providing an implant comprising a biocompatible metal surface, contacting the biocompatible metal surface with an electrolyte solution, and (c) passing a current through the electrolyte solution between a cathode and an anode which are in contact with the electrolyte solution. The method causes micro pits to be formed, distributed over the surface of the implant.

## Description

This invention relates to a method of preparing an implant having a pitted surface.

Joint replacement, or arthroplasty, is a surgical procedure in which the diseased parts of a j oint are removed and replaced with new, artificial parts. Metals and metal alloys are commonly used in making medical or orthopaedic implants, such as artificial hip joints. The implant comprises a bearing material which articulates against a hard counterface such as a metal, ceramic, or polymer counterpart. In recent years, it has become increasingly apparent that tissue necrosis and osteolysis at the interface of the orthopaedic implant and the host bone are primary contributors to the long-term loosening failure of prosthetic joints. It is generally accepted by orthopaedic surgeons and biomaterials scientists that this tissue necrosis and osteolysis is due, at least in part, to the presence of microscopic particles of metal or metal alloys produced during the wear of the metal components. The reaction of the body, e.g., immune response, to these particles includes inflammation and deterioration of the tissues, particularly the bone to which the orthopaedic implant is anchored. Eventually, the orthopaedic implant becomes painful and/or loose and must be revised and/or replaced.

Healthy animal joints have an extremely low coefficient of friction and little wear due to cartilage and natural lubricants, e.g. body fluids, formed between joint components. Such minimal friction is difficult to achieve with engineered artificial joints. One problem that contributes to increased wear in artificial joints is a lack of sufficient lubrication between the contact surfaces of the implant. Additionally, the resulting friction between the surfaces produces wear debris that is an important contributor to pathologic tissue response.

Attempts have been made to reduce metal implant wear and the associated particulate debris of metal implant wear. Attempts have been made to reduce wear by polishing the biocompatible metal surface of the implant with carbide containing polishing tools. However, with polishing, inclusions may form from the sharp edges of polish lines or from the high spots of carbides found on the metal implant surface. During the break-in period, that is after the implant has been inserted, inclusions may fall off the implant surface, or carbide inclusions may be released from the implant surface.

Further with hip replacement systems, for example, it has been attempted to reduce metal wear by reducing the diametrical clearance between femoral heads and inserts, however, this requires strict manufacturing control to maintain the tolerances. But this manufacturing control cannot stop the inclusions in the wear interface of the metal implants from dropping off and into the body fluid.

Another proposed method of reducing metal wear is to create grooves on the metal implant surface. However, as with the other methods, hard inclusions may still drop off the surface and cause wear of the implant components.

The present invention provides a method for producing a medical implant or medical implant part. The method comprises subjecting a biocompatible metal or biocompatible metal alloy implant or implant part to electrochemical etching so as to remove inclusions and distribute micro pits on the surface of the implant.

The invention provides, in an embodiment, a method of preparing an implant comprising providing an implant comprising a biocompatible metal surface and subjecting the biocompatible metal surface of the implant to electrochemical etching by contacting the biocompatible metal surface with an electrolyte solution through which a current is passed between a cathode and an anode in contact with the electrolyte solution.

The present invention seeks to reduce or eliminate a disadvantage that often accompanies metallic implants produced by a casting process. During the casting process of metallic implant parts, certain undesirable compounds such as carbides are formed on the surface as inclusions. For example, in the case of implant made from a cobalt chromium molybdenum alloy, these are M7C3 carbides. The inclusions tend to project above the surface of the implant head and the socket and, after being worn away, may pass as tiny particles into the joint cavity between the moving parts of the implant.

The electrochemical etching method in accordance with the invention creates a biocompatible metal surface that contains micropitting by removing inclusions present on the implant surface. This micropitted surface provides a more corrosion resistant surface. The micropitting on the metal surface may also provide spaces for the serum lubricant, thereby enhancing lubrication and reducing wear.

Electrochemical treatment of the biocompatible metal surface creates a metal surface that mimics the surface morphology of implant surfaces, which have been subjected to a break-in period. During the break-in period, inclusions are removed from a new implant surface. With this break-in period achieved, a favourable lubrication state can be established.

The medical implant device or component thereof can be any suitable medical implant device or component thereof. Suitable medical implant devices and components thereof include, but not limited to, orthopaedic prostheses for the hip, knee, ankle, shoulder, elbow, and spine. Exemplary medical implant devices include a full or partial knee arthroplasty prosthesis, full or partial hip arthroplasty prosthesis, full or partial elbow arthroplasty prosthesis, full or partial wrist arthroplasty prosthesis, full or partial shoulder arthroplasty prosthesis, full or partial ankle arthroplasty prosthesis, and full or partial articulating spinal segment arthroplasty prosthesis. Exemplary components of medical implant devices include a femoral component (e.g., for replacing one or more femoral condyles) or a tibial component (e.g., for replacing at least a portion of a proximal tibial plateau) of a knee prosthesis (e.g., a uni-compartmental or total knee arthroplasty prosthesis), a femoral component (e.g., for replacing at least the proximal portion or head of the femur) or an acetabular cup (e.g., for replacing the hip bone's femoral socket) of a hip prosthesis, a humeral component (e.g., for replacing the distal portion of the humerus) or an ulnar component (e.g., for replacing the proximal portion of the ulna) of an elbow prosthesis, a metacarpal component (for replacing at least a portion of one or more metacarpal bones) or radial component (for replacing the distal portion of the radius) of a wrist prosthesis, a humeral component (e.g., for replacing the proximal portion or head of the humerus) or glenoid component (e.g., for replacing the glenoid or socket portion of the scapula) of a shoulder prosthesis, a tibial component (e.g., for replacing the distal portion of the tibia) or talar component (e.g., for replacing the proximal portion of the talus) of an ankle prosthesis, and an endplate component (e.g., for contacting the superior or inferior portion of a cervical, lumbar or thoracic vertebra) or spacer component (e.g. for insertion between endplate components) of a vertebral disc prosthesis.

The metal substrate can be any suitable metal substrate other than steel. Unless otherwise indicated herein, the term "metal" refers to pure metals and metal alloys. The "metal substrate" can be the entire, or nearly the entire, structure that substantially forms the medical implant device or component thereof; or the "metal substrate" can be a portion of the structure that substantially forms the medical implant device or component thereof, with the remainder of the structure that substantially forms the medical implant device or component thereof comprising other material. When the metal substrate is a portion of the structure that substantially forms the medical implant device or component thereof, the metal substrate can be the entire surface of, or a portion of the surface of, the structure that substantially forms the medical implant device or component thereof.

The metal substrate can comprise, consist essentially of, or consist of any suitable metal, desirably a biocompatible metal. Desirable metals include metals with suitable mechanical properties for use in joint replacement prostheses. The metal preferably does not readily corrode in a patient into which the medical implant device or component thereof is intended to be placed, and preferably possesses appropriate strength and fatigue characteristics. Exemplary preferred metal substrates include cobalt, cobalt alloys, titanium, titanium alloys, and mixtures of these. Suitable cobalt-chromium alloys include, but are not limited to, the cast, forged, and wrought cobalt-28-chromium-6-molybdenum (Co28Cr6Mo) alloys described in, for example, ASTM Standards F75-01, F799-02, and F1537-00, respectively. Suitable titanium-aluminum alloys include, but are not limited to, the titanium-3-aluminum-2.5-vanadium alloy (T1-3A1-2.5V) described in, for example, ASTM Standard F2146-01 and the titanium-6-aluminum-4-vanadium (Ti-6Al-4V) alloy described in, for example, ASTM Standard F136-02a. ASTM standards are available in print or electronic media from ASTM International (West Conshohocken, PA).

In a preferred embodiment of the inventive method, the implant is exposed to an electrolyte solution. The electrolyte solution used in the inventive method comprises an electrolyte selected from the group consisting of a water soluble inorganic compound, a water soluble organic compound, an acid, a base, a water soluble oxidizer, an alcohol, a glycol, a glycol ether, an amine, an amide, a pyrrolidone, and mixtures thereof. Typically, the electrolyte is present in the electrolyte solution at a concentration of about 0.05 M to about 5 M, preferably a concentration of about 0.05 M to about 3 M, and more preferably at a concentration of about 0.1 M to about 1 M.

Any suitable water soluble inorganic compound can be used to form the electrolyte solution. Suitable water soluble inorganic compounds include salts of Group Ia, Group IIa, transition metals, and mixtures thereof. Examples of suitable metals cations include; lithium, sodium, potassium, magnesium, and calcium. In accordance with embodiments of the invention, the water soluble inorganic compound may be selected from the group consisting of chlorides, such as sodium chloride (NaCl), potassium chloride (KCl), calcium chloride (CaCl₂), magnesium chloride (MgCl₂), and ammonium chloride (NH₄Cl); phosphates, such as dibasic sodium phosphate (Na₂HPO₄), monobasic sodium phosphate (NaH₂PO₄), monobasic potassium phosphate (KH₂PO₄), and dibasic potassium phosphate (K2HPO₄); sulphates such as sodium sulphate (Na₂SO₄), potassium sulphate (K₂SO₄), and ammonium sulphate ((NH₄)₂SO₄); nitrates such as sodium nitrate (NaNO₃), potassium nitrate (KNO₃), ammonium nitrate (NH₄NO₃), and potassium nitrite (KNO₂); and mixtures thereof. Typically, the water soluble inorganic compound is present in the electrolyte solution at a concentration of about 0.05 M to about 5 M, preferably a concentration of about 0.05 M to about 3 M, and more preferably at a concentration of about 0.1 M to about 1 M.

Any suitable water soluble organic compound can be used in preparing the electrolyte solution. Suitable water soluble organic compounds include sugars and other carbohydrates. Examples of suitable organic compounds include: tetroses such as erythrose, threose, and erythrulose; pentoses, such as ribose, arabinose, xylose, lyxose, ribulose, and xylulose; hexoses, such as allose, altrose, glucose, mannose, gulose, idose, galactose, talose, psiscose, fructose, sorbose, and tagatose; disaccharides, such as sucrose, lactose, maltose, trehalose, and cellobiose; oligosaccharides; polysaccharides; and mixtures thereof. In a preferred embodiment, the water soluble organic compound is glucose. Typically, the water soluble organic compound is present in the electrolyte solution at a concentration of about 0.05 M to about 5 M, preferably a concentration of about 0.05 M to about 3 M, and more preferably at a concentration of about 0.1 M to about 1 M.

Any suitable acid may be used with the invention. Suitable acids include mineral acids and organic acids. Suitable mineral acids, for example, include hydrochloric acid, sulphuric acid, nitric acid, phosphoric acid, and mixtures thereof. Suitable organic acids, for example, include formic acid, acetic acid, citric acid, tartaric acid, oxalic acid, malonic acid, glutaric acid, adipic acid, glucuronic acid, glycollic acid, chloroacetic acid, dichloroacetic acid, trichloroacetic acid, fluoroacetic acid, bromoacetic acid, nitroacetic acid, propionic acid, butyric acid, chlorobutyric acid, phenols, and mixtures thereof. In a preferred embodiment the acid is hydrochloric acid, sulphuric acid, or nitric acid. Typically, the acid is present in the electrolyte solution at a concentration of about 0.05 M to about 5 M, preferably a concentration of about 0.05 M to about 3 M, and more preferably at a concentration of about 0.1 M to about 1 M.

Any suitable base may be used with the invention. Suitable bases, for example, include alkali bases (wherein the alkali metal is lithium, sodium, potassium, rubidium, and/or cesium) such as the hydroxides or carbonates; alkaline earth bases (wherein the alkaline earth metal is beryllium, magnesium, calcium, strontium, barium, and/or radium) such as the hydroxides and carbonates; organic bases, such as for example, ammonium hydroxide, amines such as primary, secondary or tertiary amines (e.g., diethylamine, triethylamine) and alkanolamines such as ethanolamine, diethanolamine, propanolamine, dipropanolamine, etc. Preferably, the base is selected from the group consisting of sodium hydroxide, potassium hydroxide, sodium carbonate, sodium bicarbonate, and mixtures thereof. Typically, the base is present in the electrolyte solution at a concentration of about 0.05 M to about 5 M, preferably a concentration of about 0.05 M to about 3 M, and more preferably at a concentration of about 0.1 M to about 1 M.

Any suitable water soluble oxidizer may be used with the invention. Suitable water soluble oxidizers, for example, include peroxides such as hydrogen peroxide, alkali or alkaline earth metal nitrates, nitrites, perchlorates, chlorates, chlorites, hypochlorites, dichromates, permanganates, persulphates, and mixtures thereof. In a preferred embodiment the water soluble oxidizer is hydrogen peroxide. Typically, the soluble oxidizer is present in the electrolyte solution at a concentration of about 0.05 M to about 5 M, preferably a concentration of about 0.05 M to about 3 M, and more preferably at a concentration of about 0.1 M to about 1 M.

In a preferred embodiment, the biocompatible metal surface to be electrochemically etched is placed in contact with the anode.

The biocompatible metal surface is exposed to the electrochemical etching for a time and under conditions sufficient to provide the biocompatible metal surface with the desired properties. Typically, the metal surface is subjected to the electrochemical etching from about 0.5 minutes to about 30 minutes, preferably from about 1 minute to about 20 minutes, and more preferably from about 2 minutes to about 10 minutes.

Typically, the metal surface is subjected to the electrochemical etching in an electrolyte solution to which is applied a current density from about 0.001 mA.cm⁻² to about 10 A.cm⁻², preferably from about 0.01 mA.cm⁻² to about 5 A.cm⁻², and more preferably from about 0.1 mA.cm⁻² to about 1 A.cm⁻².

Typically, the biocompatible metal surface has been subjected to the electrochemical etching in which the temperature of the electrolyte solution is from about 4°C to about 80°C, preferably from about 10°C to about 60°C, and more preferably at about room temperature.

Before the etching process begins, the substrate surface can be cleaned using typical cleaning procedures, such as degreasing with detergent or an alkaline solution. The substrate surface may be degreased by ultrasonic cleaning in detergent, followed by ultrasonic cleaning in operating room water and drying. The cleaned metal surface is then exposed to a suitable volume of the electrochemical etching solution in a container or bath. The volume of the etching solution depends on the surface area of the substrate for which etching is desired. In some instances, the entire surface of the implant will be etched, and thus the volume of the etching solution should be sufficient to cover the entire implant. In other applications, only a portion of the implant will be etched and only a desired portion of the implant need be exposed to the etching solution. One skilled in the art will readily appreciate the volume of etching solution that is required for a given etching procedure.

The electrochemical etching creates a surface with dimples, micro pits, etched spots, peaks, valleys, and/or pores. The surface morphology can be expressed in any suitable manner. In a preferred embodiment of the invention the biocompatible metal surface has a more negative Rₛₖ value after the electrochemical etching than before the electrochemical etching. Rₛₖ (Skew) is a measure of the symmetry of a profile about a mean line. A negative Rₛₖ value indicates a predominance of valleys or indentations in the implant surface, while a positive Rₛₖ value indicates a predominance of peaks in the implant surface. Rₛₖ is further explained in ISO 4287:1997. ISO standards are available in print or electronic media from the International Organization for Standardization (Geneva, Switzerland). For example, the biomedical implant has an Rₛₖ value from about -0.001 to about -15, preferably from about -0.01 to about -10, and more preferably from about -0.1 to about -5.

The invention provides a biomedical implant, e.g., an orthopaedic implant, which has at least on a portion thereof, a textured surface formed, for example, of a plurality of discrete or indiscrete indentations, e.g., dimples, micro pits, etched spots, peaks, valleys, and/or pores. The micro pits of the textured surface can be in the nanometre to micrometre size range in both diameter and depth. The micro pits can have a diameter in the range of about 100 nm to 15 µm, preferably from 100 nm to 10 µm, more preferably from 100 nm to 5 µm. The depths of the micro pits may vary; typically, the micro pits an have depths of less than about 10 µm, preferably less than about 8 µm.

### EXAMPLE

This example demonstrates the electrochemical etching process of the invention. A CoCrMo surface is electrochemically etched in an electrolyte solution of 0.5 N HNO3 for 10 minutes with a current density of 0.2 A.cm⁻². The resulting implant surface includes a surface with micro pits and is substantially free of inclusions, for example, carbides.

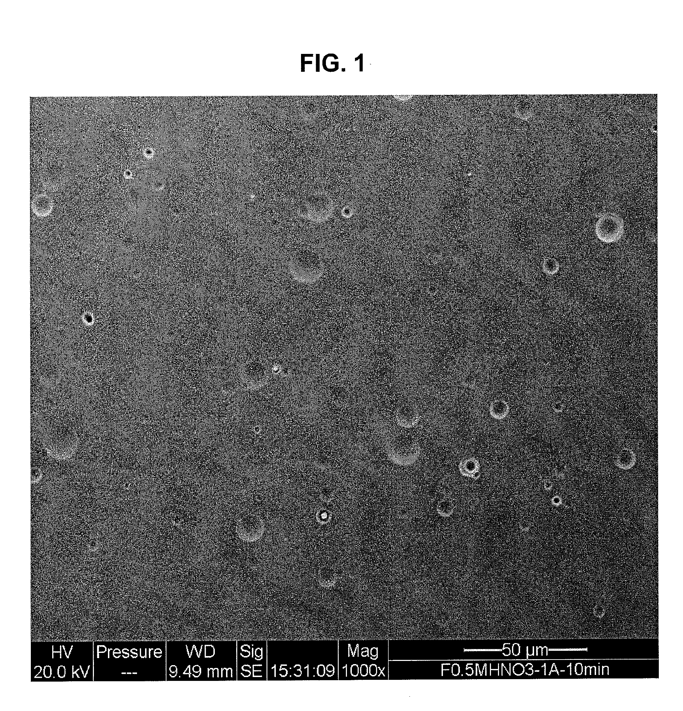

FIG. 1 is a scanning electron microscopy micrograph (1000 × magnification) of the surface of a cobalt chromium molybdenum surface made according to an embodiment of the invention.

## Claims

1. A method of preparing an implant, comprising: (a) providing an implant comprising a biocompatible metal surface, (b) contacting the biocompatible metal surface with an electrolyte solution, and (c) passing a current through the electrolyte solution between a cathode and an anode which are in contact with the electrolyte solution, to form micro pits distributed over the surface of the implant.

2. The method of claim 1, in which the biocompatible metal surface comprises an alloy, preferably a cobalt-chromium-molybdenum alloy.

3. The method of claim 1, in which the electrolyte solution comprises an electrolyte selected from the group consisting of a soluble inorganic compound, a soluble organic compound, an acid, a base, hydrogen peroxide, ethanol, and mixtures thereof.

4. The method of claim 3, in which the electrolyte is present in the electrolyte solution at a concentration of about 0.05 M to about 5 M, preferably about 0.1 M to about 1 M.

5. The method of claim 3, in which the electrolyte is a soluble inorganic compound selected from the group consisting of sodium chloride (NaCl), potassium chloride (KCl), calcium chloride (CaCl₂), magnesium chloride (MgCl₂), ammonium chloride (NH₄Cl), dibasic sodium phosphate (Na₂HPO₄), monobasic sodium phosphate (NaH₂PO₄), monobasic potassium phosphate (KH₂PO₄), dibasic potassium phosphate (K₂HPO₄), sodium sulphate (Na₂SO₄), potassium sulphate (K₂SO₄), ammonium sulphate ((NH₄)₂SO₄) sodium nitrate (NaNO₃), potassium nitrate (KNO₃), ammonium nitrate (NH₄NO₃), potassium nitrite (KNO₂), and mixtures thereof.

6. The method of claim 3, in which the electrolyte is an organic compound, preferably comprising at least one a sugar.

7. The method of claim 3, in which the electrolyte is an acid selected from the group consisting of hydrochloric acid, sulphuric acid, nitric acid, phosphoric acid, acetic acid, citric acid, and mixtures thereof.

8. The method of claim 3, in which the electrolyte is a base selected from the group consisting of sodium hydroxide, potassium hydroxide, sodium carbonate, sodium bicarbonate, and mixtures thereof.

9. The method of claim 1, in which the biocompatible metal surface to be etched is also in contact with the anode.

10. The method of claim 1, in which the biocompatible metal surface is subjected to the electrochemical etching for a period from about 0.5 minutes to about 30 minutes, preferably from about 2 minutes to about 20 minutes.

11. The method of claim 1, in which the biocompatible metal surface is subjected to the electrochemical etching in which the current density is from about 0.001 mA.cm⁻² to about 10 A.cm⁻², preferably from about 0.01 A.cm⁻² to about 1 A.cm⁻².

12. The method of claim 1, in which the biocompatible metal surface is subjected to the electrochemical etching in which the temperature of the electrolyte solution is from about 4°C to about 80°C, preferably from about 15°C to about 25°C.

13. The method of claim 12, in which the biocompatible metal surface has a more negative Rₛₖ value after the electrochemical etching than before the electrochemical etching.
